# EUROPEAN PATENT APPLICATION

(11) **EP 1 454 524 A1**
(43) Date of publication of application: **08.09.2004**
(21) Application number: 02768013.1
(22) Date of filing: 24.09.2002
(51) Int. Cl.: A01K 67/027, C12N 15/00

(54) **HCV GENE TRANSGENIC ANIMAL**

(30) Priority: 30.10.2001 JP 2001332475
(71) Applicant: Tokyo Metropolitan Organization for Medical Research, Shinjuku-ku, Tokyo 163-8001 (JP); CHUGAI SEIYAKU KABUSHIKI KAISHA, Tokyo, 115-8543 (JP)
(72) Inventor: KOHARA, Michinori, Kita-ku, Tokyo 114-0014 (JP); SUZUKI, Hiroshi, Gotenba-shi, Shizuoka 412-8513 (JP); UEDA, Otoya, Gotenba-shi, Shizuoka 412-8513 (JP); JISHAGE, Kou-ichi, Gotenba-shi, Shizuoka 412-8513 (JP); KATSUME, Asao, Gotenba-shi, Shizuoka 412-8513 (JP)
(74) Representative: Soames, Candida Jane
(86) International application number: PCT/JP2002/009767
(87) International publication number: WO 2003/037081

(57) **Abstract**

The present invention provides a transgenic animal expressing the full-length human hepatitis C virus (HCV) for the purpose of constructing a system for screening for a remedy for human hepatitis C.

The present invention is a transgenic hepatitis C model animal, which has the full-length DNA of the hepatitis C virus incorporated therein and can express the full-length HCV gene, and a method of generating a hepatitis C model animal carrying the full-length HCV gene, which comprises introducing a vector containing the full-length DNA of hepatitis C virus into an ES cell, and causing the ES cells to undergo ontogenesis in a pseudo-parent.

## Description

### TECHNICAL FIELD

The present invention relates to a transgenic animal expressing the full length of the human hepatitis C virus (HCV) for the purpose of constructing a system for screening for a remedy for human hepatitis C.

### BACKGROUND ART

There have been many attempts to generate a model animal relating to the onset of hepatitis by introducing a hepatitis C virus (hereinafter referred to as "HCV") gene into a small animal such as a mouse, and causing the introduced gene to be expressed (C. Pasquinelli et al., Abstract book of 2nd international meeting on hepatitis C virus and related viruses (July 31-August 5, 1994 San Diego, USA); C. Pasquinelli et al., Abstract book of 3rd international meeting on hepatitis C virus and related viruses (August 28, September 3, 1995 Gold coast, Australia); Kazuhiko Koike et al., J. General Virology, 76, pp. 3031-3038, 1995; T. Kato, Arch Virol, 141, pp. 951-958, 1996). Similarly to HCV, in the case of poliomyelitis virus having infectivity to only humans and chimpanzees, a transgenic mouse having a receptor gene introduced thereto has been developed, and is utilized in a system for evaluating vaccines. However, much about HCV is unknown, such as the receptor gene and the intracellular behavior of the virus after infection. Unlike many other genes, incorporation of an HCV gene into a mouse individual is difficult. In addition, even in the case of successful incorporation of the HCV gene, production of an HCV protein has not been observed in most cases. Furthermore, even in extremely rare cases wherein production of the HCV protein has been observed, it was found that mice have become immunologiclally tolerant due to the fact that protein production had begun from the fetal period, and thus none of them have been found to present typical hepatitis symptoms after birth. Such mice have been unable to be hepatitis models.

We have succeeded in causing a mouse to express pathological conditions resembling those of human hepatitis C by introducing a vector, which has been previously constructed to cause the expression of a partial cDNA derived from HCV by switching expression into a fertilized mouse egg by microinjection (JP Patent Publication (Kokai) No. 10-84813 A (1998)). Here, switching expression refers to an expression system whereby a specific gene can be expressed at a desired time, and specifically whereby an HCV-derived protein can be produced at a stage where an animal has grown to a certain degree. Thus, the problem of immunological tolerance that has been an issue in hepatitis C model animals could be avoided.

However, a partial HCV gene is expressed in this invention, and there have been no reports that the full-length gene has been expressed.

There has been an attempt to generate an HCV-expressing model animal by introducing the full-length HCV gene into a mouse (Matsuda et al., Jpn. J. Cancer Res., 89, 150-158, February 1998). However, expression control as described above was not performed, and no data showing the expression of the full-length HCV in the progeny mice has been obtained.

In the meantime, we have succeeded in constructing a vector that is capable of causing the expression of the full-length HCV gene (JP Patent publication (Kokai) No. 2000-152793 A) by switching expression. However, at the time of the establishment of this vector, there have been no reports that a transgenic animal stably expressing the gene has been generated.

### SUMMARY OF THE INVENTION

An object of the present invention is to provide a hepatitis C model animal expressing the full-length HCV gene, and a method of generating the model animal.

In view of the above problems, we have intensively studied the generation of a hepatitis C model animal. Thus, we have completed the present invention by discovering that a transgenic animal line stably expressing an HCV gene can be generated by introducing a vector constructed to cause the expression of a full-length HCV cDNA by switching expression into an animal through the use of ES cell.

That is, the present invention is summarized by the following subject matters.
(1) A transgenic hepatitis C model animal, having the full-length DNA of the hepatitis C virus incorporated therein, and being capable of expressing the full-length HCV gene.
(2) The hepatitis C model animal of (1), wherein the animal is a mouse.
(3) The hepatitis C model animal of (1) or (2), wherein the full-length DNA of the hepatitis C virus is introduced so that it is expressed by switching expression.
(4) The hepatitis C model animal of (3), wherein a means to cause the expression of the full-length DNA of hepatitis C virus by switching expression comprises causing a stuffer gene flanked by loxP sequences to be present between the full-length DNA of the hepatitis C virus and a promoter.
(5) The hepatitis C model animal of any one of (1) to (4), which is a mammal obtained by developing ontogenetically ES cell (embryonic stem cell) wherein the full-length DNA of the hepatitis C virus is introduced through a chimeric embryo, or the offsprings thereof, and which carries the above full-length HCV gene in the chromosome of the somatic cell and the germ cell.
(6) A method of generating a hepatitis C model animal carrying a full-length HCV gene, comprising introducing a vector containing the full-length DNA of hepatitis C virus into ES cell, and developing ontogenetically the ES cell through a chimeric embryo.
(7) The generation method of (6), wherein the animal is a mouse.
(8) The generation method of (6) or (7), wherein the full-length DNA of hepatitis C virus is introduced so that it is expressed by switching expression.
(9) The generation method of (8), wherein a means to cause the expression of the full-length DNA of the hepatitis C virus by switching expression comprises causing a stuffer gene flanked by loxP sequences to be present between the full-length DNA of the hepatitis C virus and a promoter.
(10) A method of screening for a remedy for a hepatitis C virus-related disease or a substance suppressing the proliferation of hepatitis C virus, comprising the following steps of:
   (a) administering a test substance to the animal of any one of (1) to (5),
   (b) measuring the hepatitis C virus level in the above animal, and
   (c) selecting a substance that reduces hepatitis C virus level compared with that in a case wherein a test substance is not administered.
(11) The hepatitis C model animal of any one of (1) to (5), wherein an immunodeficient state is introduced.

The method of generating a transgenic animal used in the present invention, which is characterized in that it comprises introducing a target gene into a totipotent cell and developing ontogenetically the totipotent cell, is considered to be particularly appropriate for the generation of transgenic mice capable of expressing a protein or the like the expression of which has been conventionally difficult. Therefore, even a protein or the like the expression of which has been difficult by transgenic mice generated by other methods can be expressed by the method of generating transgenic animals used in the present invention. The phrase used herein, "a protein or the like the expression of which is difficult" refers to a protein or the like for which the expression of the whole or a part thereof cannot be confirmed in transgenic animals because of its low expression amounts, or a protein or the like whose expression is too strong to cause death in transgenic animals generated by other methods.

The present invention is a pathological model animal wherein the full-length HCV DNA gene is introduced so that it can be expressed. A line of this model animal has been established, so that it can transmit a transgene to its offspring.

### 1. Construction of a vector containing the full-length HCV gene

The vector to be used in generating the model animal of the present invention is preferably a vector that is so constructed that both ends of HCV gene can be precisely and uniformly transcribed.

Here, the phrase "both ends can be precisely transcribed" means that RNA produced from DNA is completely the same as the original viral genomic RNA, or that there is only a slight difference in nucleotide sequence between the two at a level that has no effect on the translation ability. In addition, the phrase "both ends can be uniformly transcribed" means that RNA having a specific nucleotide sequence can be produced with a level of certain reproducibility.

A method that can be exemplified to precisely and uniformly transcribe both ends of RNA virus gene is, but is not limited to, a method that involves locating a DNA encoding ribozyme that cleaves by self-processing upstream of the 5' end and downstream of the 3' end of a DNA encoding the HCV gene. An example of such a DNA is a DNA encoding a hammerhead ribozyme (JP Patent Publication (Kokai) No. 2000-152793 A).

A vector (p5'-3'RBZ) containing the full-length HCV gene can be prepared by cloning a DNA fragment containing two of the above-described DNA encoding ribozyme and the HCV gene by PCR or the like, and inserting the DNA fragment into a vector containing an appropriate promoter and a terminator according to the method described in JP Patent Publication (Kokai) No. 2000-152793 A.

The vector of the present invention may be a vector that drives gene expression immediately after it is transferred into a host cell, but is preferably a vector that begins expression only when subjected to a specific treatment. As a means to initiate expression by a specific treatment, examples include a means using a promoter that is not recognized by the RNA polymerase of a host cell, a means using a Cre/loxP expression system (Nat Sternberg et al., J. Molecular Biology 150, pp. 467-486, JP Patent Publication (Kokai) No. 10-84813 A (1998)) and the like can be exemplified. In the former means, expression of a target gene can be initiated by expressing RNA polymerase capable of recognizing a promoter within a vector in a host cell. In the latter means, expression of a target gene can be initiated by expressing Cre enzyme in a host cell.

The Cre/loxP expression system consists of an inserted gene (Stuffer, a gene that is present between a promoter and a target gene, suppressing the expression of the gene) flanked by 2 loxP sequences and P1 phage CreDNA recombinant enzyme (hereinafter, simply referred to as "Cre") that removes the inserted gene together with one loxP sequence. The system enables expression of a target gene at any period by causing Cre to act. Stuffer is not limited to a specific gene. An example of Stuffer is a neomycin resistance gene. The loxP sequence is a 34-bp long DNA derived from the *Escherichia coli* P1 phage gene. Cre is a DNA recombinant enzyme with an approximate molecular weight of 38 kD derived from *Escherichia coli* P1 phage. For example, Cre can be caused to act by infection with an adenovirus expressing Cre. By causing the expression of cDNA derived from HCV to take place by switching expression, it is possible for the animal to produce an HCV-derived protein at a stage when the animal has grown to some extent.

The vector (pCALN/pBR) having a function to cause expression by switching expression and containing a promoter and loxP sequences can be prepared according to the method described in JP Patent Publication (Kokai) No. 10-84813 A (1998). *Escherichia coli* wherein the pCALN/pBR had been introduced was deposited with the National Institute of Advanced Industrial Science and Technology, International Patent Organism Depositary (Central 6, 1-1-1 Higashi, Tsukuba, Ibaraki, Japan) under the accession No. FERM P-15753 on July 22, 1997.

By the use of the above vector containing the full-length HCV gene and the vector having a function to cause expression by switching expression and containing a promoter and loxP sequences, a vector (pCALN/HCV RBZ) that is directly used for generating a model animal having the full-length HCV gene of the present invention can be prepared according to the method described in JP Patent Publication (Kokai) No. 2000-152793 A. *Escherichia coli* wherein the pCALN/HCV RBZ had been introduced was deposited with the National Institute of Advanced Industrial Science and Technology, International Patent Organism Depositary (Central 6, 1-1-1 Higashi, Tsukuba, Ibaraki, Japan) under the accession No. FERM BP-6763 on October 31, 1997.

### 2. Generation of transgenic animals expressing the full-length HCV gene

A transgenic animal can be generated according to the method in Pro. Natl. Acad. Sci. USA 77:7380-7384, 1980 or the like by introducing the above transgene into mammalian totipotent cells, selecting cells having the transgene incorporated in the chromosome, and then causing the cells to develop into individuals. As the totipotent cell to be developed into an individual, it is desirable to previously select a totipotent cell having a desired expression degree of a target protein. Preferable examples of a mammal include rodents such as mice, rats and hamsters. Among them, mice, for which many inbred lines have been generated and for which techniques for culturing fertilized eggs, in vitro fertilization and the like have been well established, are particularly preferred. Technically all animal species excluding humans may be targeted. Examples of a totipotent cell to which a gene is introduced include cultured cells such as ES cells having pluripotency in addition to fertilized eggs and early embryos. When generation efficiency of transgenic animal individuals and transmission efficiency of a transgene to the following generation are taken into consideration, the use of ES cells is desired. When ES cell is injected to a blastocyst, it is mixed with the inner cell mass of a host embryo so as to contribute to the formation of a mouse embryo and fetus, so that a chimeric mouse can be generated. In addition, a mouse, essentially a fetus entirely derived from ES cells may be generated. When ES cells contribute to the formation of primordial germ cells that will form oocytes or sperm in the future within a chimeric mouse, a germ line chimera can be generated. By crossing the chimeric mice, ES cell-derived mouse individuals can be obtained. As a method for introducing a gene into a cultured cell, known as electroporaion method, liposome method, calcium phosphate method or the like can be utilized. The electroporation method is preferred.

In some ES cells, in which the full-length HCV gene has been introduced, a transgene is introduced into the cell chromosome. When a marker gene is utilized in a gene transfer vector, a cell wherein a desired gene has been introduced would obtain the marker gene. Thus, selection can be performed using the marker gene as an indicator. For example, when a drug resistance gene is used as a marker gene, cells to which desired genes have been introduced can be selected by culturing cells after vector introduction in the presence of the drug at a lethal concentration.

The mammals of the present invention can be generated by assembling the above mammalian cells with early embryos or injecting the cells into the blastocoel to obtain chimeric embryos, transferring the chimeric embryos to the uteri of pseudopregnant females, and then allowing development into individuals. When ES cells are injected into host embryos, a chimeric embryo can be prepared. Blastocysts for injection can be obtained by flushing the uterus of pregnant females. To make it possible to test after the generation of individuals whether or not the cell injected into an embryo has been incorporated into the embryo during development and differentiation, it is desirable to select a strain, from which blastcysts are obtained, so that the external characteristics (e.g., coat color) of the generated individuals differ between parts derived from injected cells and parts derived from the blastcyst. Animals having the full-length HCV DNA are selected among the animals obtained, and then animals expressing HCV-derived DNA by switching expression are selected, so that the hepatitis C model animal of the present invention can be obtained. Furthermore, when a germ cell of a chimeric animal is derived from injected cells, the hepatitis C model animal of the present invention can also be obtained by breeding the chimeric animal with an animal of an appropriate strain of the same species to produce progeny.

PCR is performed using DNA extracted from the thus obtained animal as a template, and primers that are prepared by synthesizing oligonucleotides corresponding to the integrated DNA. If HCV cDNA had been integrated, amplified HCV DNA would be detected, but if no HCV DNA had been introduced, amplified HCV DNA would not be detected. Furthermore, whether HCV DNA is expressed by switching expression may be confirmed by removing a sequence inhibiting the expression of HCV DNA and examining whether the protein corresponding to the DNA is produced in vitro or in vivo. Whether or not a protein corresponding to HCV DNA is produced can be determined by the Western blot method, the fluorescent antibody staining method or the like.

Since HCV DNA is expressed by switching expression, the hepatitis C model animal of the present invention does not develop hepatitis in its original state. To cause the onset of hepatitis, it is necessary to remove a sequence suppressing the expression of the DNA. To remove such a suppression sequence, a DNA recombinase may be used. For example, if a suppression sequence is the loxP sequence, AxNCre adenovirus, which expresses Cre, the enzyme removing the loxP sequence, may be allowed to infect. Cre and AxNCre can be prepared according to the description in Yumi Kanegawa et al., Nucl. Acids Res. 23, 19, 38 16-21, 1995.

Animals wherein the HCV gene is integrated in a part of a chromosome so that it can be stably expressed can be efficiently generated by crossing individuals as founders, for which the presence of a transgene has been confirmed.

Furthermore, the method of generating transgenic animals expressing the HCV gene of the present invention can also be applied not only to the full-length HCV gene, but also to a partial HCV gene.

In the present invention, for the purpose of expressing HCV in greater quantities, the transgenic animal expressing the HCV gene of the present invention may be further treated.

For example, to further increase the HCV expression level in the transgenic animal expressing the HCV gene of the present invention, immune functions of the transgenic animal of the present invention can be hindered or suppressed to put the animal in an immunodeficient state. As a specific example, when the Cre/loxP expression system is used, it is conceivable that cells infected with Cre-expressing adenovirus, which have been administered to induce HCV expression, would be eliminated by immunocytes such as CD8 antigen-positive cytotoxic T cells. In such a case, if the transgenic animal of the present invention is in an immunodeficient state, it is considered that elimination of cells infected with Cre-expressing adenovirus would be difficult, and HCV can be efficiently expressed. Examples of the transgenic animal of the present invention in an immunodeficient state are not specifically limited, as long as it is an animal wherein all or some of its immune functions are hindered or suppressed. Examples of a method of generating such an immunodeficient transgenic animal include, but are not limited to, a method that involves crossing the transgenic animal of the present invention with an immunodeficient animal to obtain such an immunodeficient animal, a method that involves administering a drug to the transgenic animal of the present invention so as to suppress immunity, and a method that involves causing immunological tolerance in the transgenic animal of the present invention.

To generate a transgenic animal in an immunodeficient state, persons skilled in the art can appropriately select an immunodeficient animal to be crossed with the transgenic animal of the present invention. When such animal is a mouse, examples of suitable mouse include nude mice, scid mice, scid-NOD mice, RAG-1KO mice, RAG-2KO mice and IRF-1KO mice.

A specific example of a drug to be administered to put the transgenic animal of the present invention in an immunodeficient state is an immunosuppresant. Examples of an immunosuppresant are not specifically limited, as long as it has an immunosuppressive effect. For example, commercially available cyclosporine, tacrolimus, steroid, anticancer agent of a certain type, an antibody having known immunosuppressive effect such as anti-CD8 antibody or the like can be used.

Immunological tolerance is a state wherein immune response is lost in an antigen-specific manner. Immunological tolerance can be induced by, for example, administering an antigen, which is a tolerogen, several times to an animal in the fetal period or immediately after birth, or by causing a gene encoding a tolerogen to be expressed in vivo in an animal. Hence, for example, to suppress elimination of adenovirus, an antigen derived from adenovirus may be used as a tolerogen.

The transgenic animal expressing the HCV gene of the present invention further includes a transgenic animal that has been further treated to express HCV in greater quantity, such as a transgenic animal wherein an immunodeficient state has been introduced.

### 3. Screening for a remedy for a hepatitis-C-virus-related disease or a substance suppressing the proliferation of the hepatitis C virus

By the use of a transgenic animal expressing the full-length HCV gene, a remedy for a hepatitis-C-virus-related disease or a substance suppressing the proliferation of the hepatitis C virus can be screened for.

A substance reducing the hepatitis C virus level, that is, a remedy for a hepatitis-C-virus-related disease or a substance suppressing the proliferation of the hepatitis C virus can be screened for by administering a test substance to the transgenic animal expressing the full-length HCV gene of the present invention, measuring the hepatitis C virus level in the animal, and then comparing the level with that in a case when no test substance was administered.

In this screening method, not only HCV itself, but also any of those directly or indirectly representing HCV levels may be measured to find HCV levels. For example, the blood viral level may be directly measured, and the HCV level may be measured using hepatitis markers such as serum biochemical values (ALT and AST) as indicaters. A simple way of measurement is direct measurement of the blood viral level. As a measurement method in this case, any known methods such as an absorbance measurement, RT-PCR, TMA-HPA or ELISA may be used.

### BRIEF DESCRIPTION OF DRAWINGS

FIG. 1 shows the construction of a vector to be used for generating the transgenic animal of the present invention. FIG. 1A shows construction steps toward p5'-3'RBZ, and FIG. 1B shows construction steps from p5'-3'RBZ to pCALN/HCV RBZ.
FIG. 2 shows tables showing the results of generating transgenic mice by the microinjection method.
FIG. 3 shows a table showing the results of gene transfer to ES cells.
FIG. 4 shows a table showing the results of generating transgenic mice through the use of ES cells.
FIG. 5 shows the expression of HCV gene in transgenic mice.

### BEST MODE OF CARRYING OUT THE INVENTION

The present invention will be further described in reference to the following examples. However, the technical scope of the present invention is not limited by these examples.

### [Example 1] DNA construct to be introduced

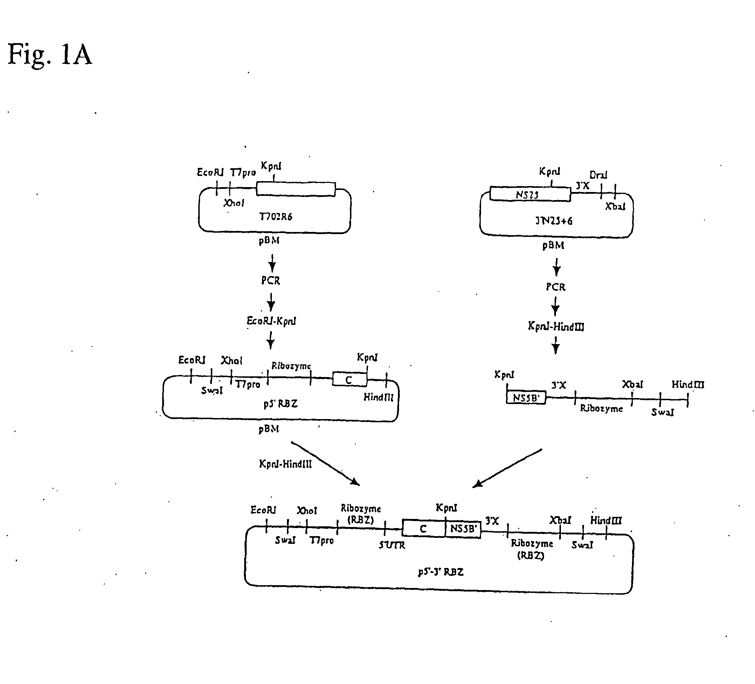

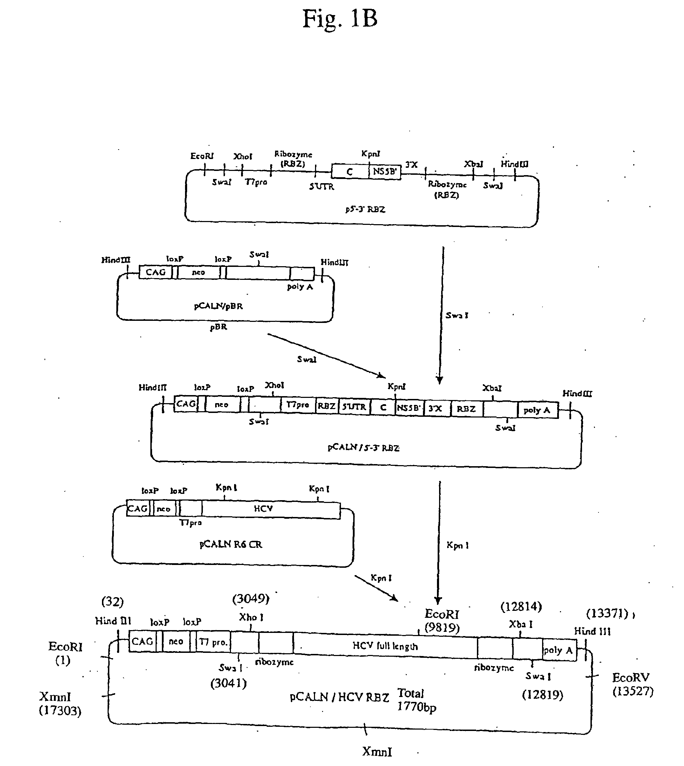

A schematic drawing of the construction of a plasmid producing infectious HCV using ribozymes is shown (Fig. 1). In Fig. I, numbers given for a plasmid at the lowermost case in the figure represent the number of nucleotides existing in a range from the position of the first nucleotide in the *Eco*R I site, which is designated to be 1, to the first nucleotide in the restriction enzyme site. *Xho* I and *Xbo* I sites other than those denoted in this figure are present. The plasmid is so constructed that a cytomegalovirus (CMV) enhancer, a chicken β actin promoter, a loxP sequence, a neomycin resistance gene, a loxP sequence, a ribozyme, the full-length HCV cDNA and a ribozyme are ligated sequentially. A DNA fragment to be introduced can be excised with *Eco*R V and *Xmn* I. A vector treated with restriction enzymes was subjected to agarose gel electrophoresis, and necessary DNA fragments were collected. The collected DNA fragments were purified using a QIAGEN Gel extraction kit, subjected to phenol/chloroform extraction followed by chloroform extraction, and then subjected to ethanol precipitation. The precipitate was washed with 70% ethanol, and then dissolved with sterile PBS.

### [Comparative example 1] Generation of transgenic mice by microinjection to fertilized eggs

### (1) Microinjection

Pronuclear fertilized eggs of C57BL/6J(B6) line or BALB/c line mice were used for microinjection of DNA fragments. Frozen fertilized eggs of the B6 line were used after thawing. Fertilized eggs of the BALB/c line were collected from the ampulla of the fallopian tubes of females that had been subjected to superovulation treatment, crossed with males of the same line, and then confirmed to have copulation plug the next morning. The collected eggs were treated with hyaluronidase to remove cumulus cells, and then washed with Whitten's (WM+EDTA) medium supplemented with 100 µM of EDTA. Pronuclear fertilized eggs were selected and then used. Fragments to be introduced were prepared at approximately 3 ng/µl by diluting with PBS, and then micro-injected into the pronuclei of the fertilized eggs using a micromanipulator. Microinjection was performed by the method described in "Method for Generating Transgenic Mice Using C57BL/6 Line Mice," Latest Technology for Gene Targeting, Experimental Medicine, separate volume, YODOSHA, 2000, pp. 190-207, Otoya UEDA, Kou-ichi JISHAGE, Hiroshi SUZUKI. On the next day, embryos that had developed to the 2-cell stage were transplanted to the fallopian tubes of recipient females. 19 days after transplantation, progeny were obtained by Caesarean section or spontaneous delivery.

The genotype analysis was carried out by Southern blot. As a probe, a neomycin resistance gene contained in a fragment to be introduced or a part of a HCV core protein gene was labeled with α[³²P] dCTP and then used.

Detection was performed for the core protein by an assay, and for NS5B protein by Western blot.

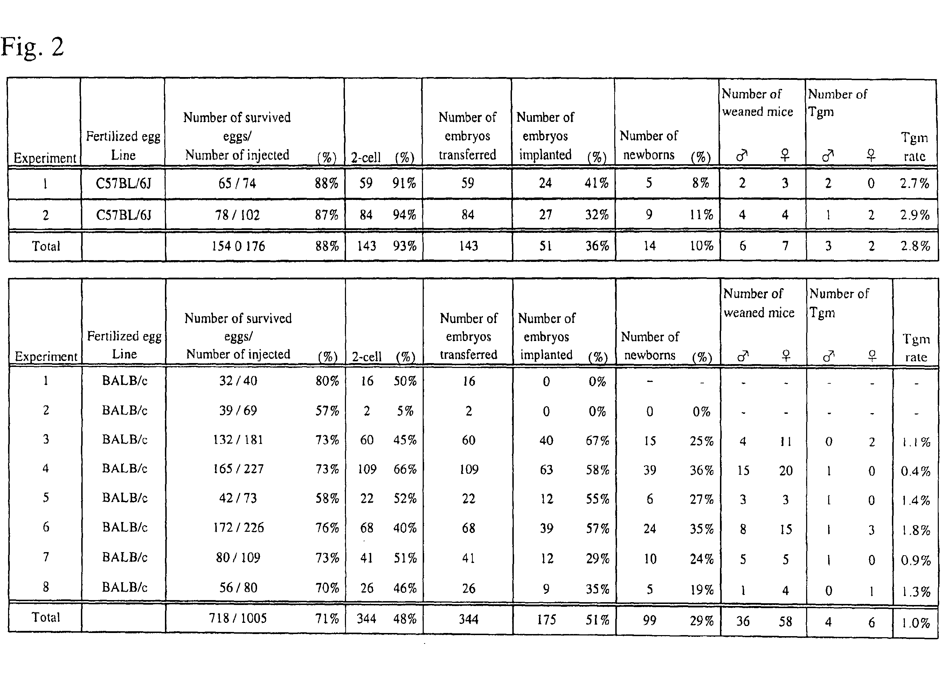

Founders obtained using the fertilized eggs of the B6 line were 5 mice (#4 male, #5 male, #8 female, #9 female and #10 male). When these founders were crossed, mice of the next generation derived from 3 (#4, #5 and #10) of these founders were obtained. In addition, the number of founders obtained using the fertilized eggs of the BALB/c line was 10 (#1-5, #1-11, #2-23, #3-5, #4-1, #4-3, #4-12, #4-21, #5-9 and #5-15). For #1-5 and #4-1 among these mice, although mice of the next generation were obtained, transmission of the transgene was not confirmed. As a result of crossing of the founders, 8 lines could be established. Gene expression was confirmed using these Tgm (transgenic mice). The analysis of gene expression involves introducing the Cre gene using an adenovirus vector, removing a neomycin resistance gene, the stuffer, to induce the expression of downstream HCV gene, and then attempting to detect the core protein and the NS5B protein. As a result, no line that could be determined to clearly express the HCV gene, was obtained. To obtain a useful line, there is a need to establish a great number of lines. However, the number of lines that can be established with the method involving microinjection into pronuclei is limited, because of its extremely low generation efficiency of 1% to 2% (Fig. 2).

### [Example 2] Gene transfer into ES cells and generation of chimeric mice

The Mouse Kit (Lexicon) was used. Culture of ES cells and screening for transformants were conducted basically according to the protocols attached to the kit. Gene transfer was performed by subjecting 40 µg of DNA fragments to be introduced to electroporation under conditions of 230 V and 500 µF. 48 hours after electroporation, the medium was exchanged with a medium supplemented with 200 µg/ml G418, and then clones to be introduced were selectively cultured. Colonies that had grown in the medium supplemented with G418 were picked up, and then allowed to proliferate in a 96-well plate. Some of the clones allowed to proliferate were subjected to genotype analysis by Southern blot. Clones for which expected signals had been observed were grown to approximately 1×10⁶. The Cre gene was introduced using an adenovirus vector (Ad/Cre) to remove the Stuffer sequence, thereby inducing the expression of the HCV gene and thus examining the expression levels of the core protein and NS5B.

Chimeric mice were generated from ES cell clones with high expression levels of the HCV core protein and the NS5B protein by the use of Ad/Cre. Blastocysts of the C57BL6(B6) strain were used as host embryos. Female mice were subjected to superovulation treatment by interperitoneally administering 5 iu. each of pregnant mare's serum gonadotropin (eCG) and human placenta chorionic gonadotrophin (hCG) every 48 hours. The female mice were crossed with males of the same strain. From females, for which copulation plug formation was confirmed the next morning, 8-cell stage to morula-stage embryos were collected on the 2.5th day of pregnancy. Blastocysts obtained after 24 hours of further culturing of the embryos were used for the injection of ES cells. A piezo-micromanipulator was used for injection. After injection, the blastocysts were transplanted into the uteri of recipient females on day 2 of pseudopregnancy. 17 days after transplantation, progeny were obtained by spontaneous delivery or Caesarean section (Kawase et al., Contemp Top Lab Anim Sci, 40, 31-34, March (2001)). At the time of weaning, the contribution ratio of ES cells to other cells was estimated based on the proportion of wild coat color derived from ES cells to others. Germ line transmission of ES cells was examined based on the coat color of progeny obtained by crossing sexually mature male chimeric mice with female mice of the B6 line.

Electroporation (EP) was performed twice. 45 G418-resistant clones were obtained by selective culture performed after the first EP. 43 of these clones were PCR-positive. However, the number of clones for which signals in the normal position were obtained by Southern blot detection was 27 out of 37 clones for which Southern blot could be performed. Clones for which signals had been observed in abnormal positions were not used for the subsequent analyses. Only the normal clones were grown and subjected to protein expression analysis.

As a result, when the detection of the NS5B protein was performed for 7 clones resulting in the expression of 400 pg/mg or more core protein, clear signals were observed for 3 (#A20, #A26 and #A41) of these clones.

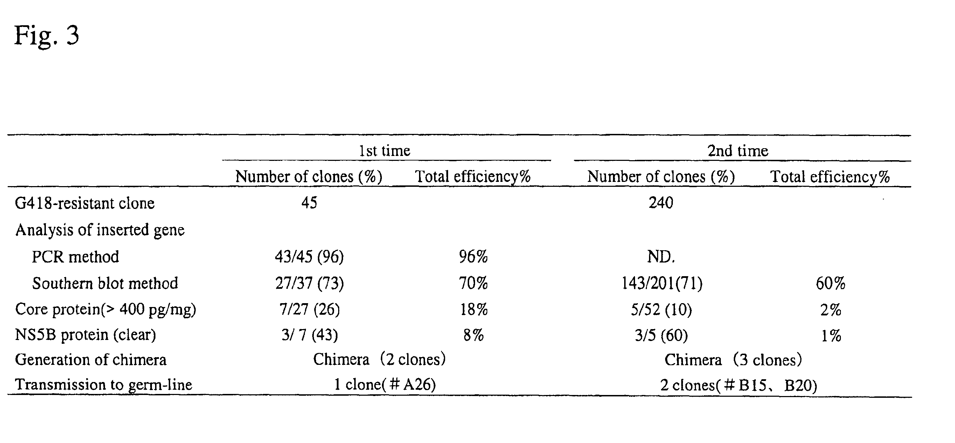

After the second EP, 240 G418-resistant clones were obtained. However, the number of clones for which signals were observed in the normal position was 143 out of 201 clones that could be analyzed by Southern blot. 52 of these clones were grown and then subjected to protein expression analysis. Among 5 clones observed to express 400 pg/mg or more core protein, NS5B protein was detected in 3 clones (#B15, #B20 and #B27) (Fig. 3).

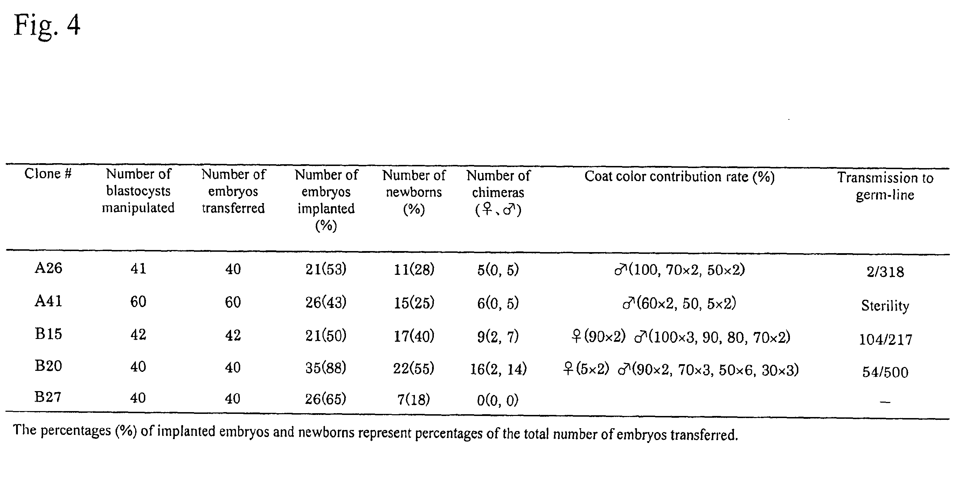

Results of generating chimeric mice are shown in this figure. Chimeric mice with coat color were obtained from 4 out of 5 clones with which generation had been conducted. For 3 of these clones, whether or not the germ line was transmitted was confirmed. With clones #A26, #B15 and #B20, progeny derived from ES cells were obtained as determined by coat color (Fig. 4).

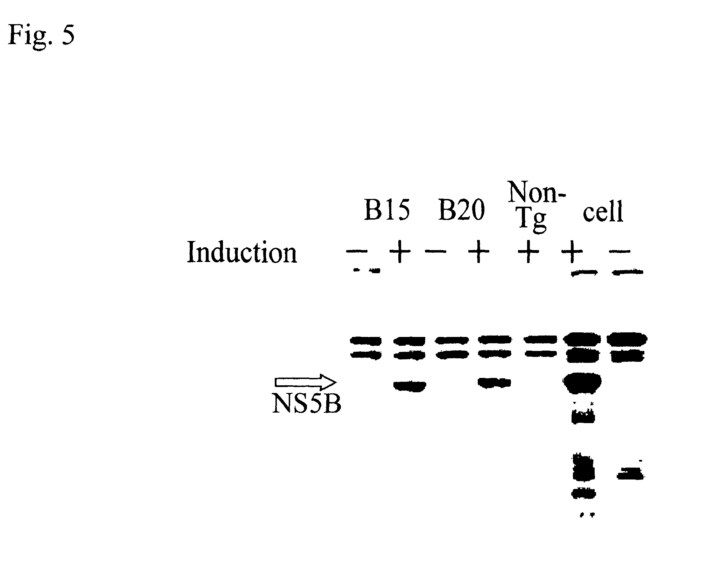

Furthermore, using 7 mice of the B15 line and 2 mice of the B20 line, primary cultures were prepared from the mouse kidney. When the induction of expression was attempted by infection with the Cre-expressing adenovirus, core protein expression at quantifiable levels was observed in both lines. At this time, only a subtle expression was observed in the A41 chimeric mouse (for which line establishment had been abandoned because of sterility) that was analyzed at the same time. Moreover, when the expression of the NS5B protein was confirmed by Western blot, NS5B was detected for both lines, revealing that the transgene was certainly functioning (Fig. 5). Furthermore, when the Cre-expressing adenovirus was directly inoculated into mice, the expression of the core protein was observed in the liver. These results suggest that the generated transgenic mouse line expressed the full-length protein. The transgenic mouse of the present invention is the world's first mouse for which the expression of an HCV protein was observed when a full-length expression unit was inserted.

### Industrial applicability

The transgenic animal of the present invention can express the full-length HCV protein, and can further transmit the full-length HCV gene to its offspring by crossing, so that it is useful in elucidating the onset mechanism of hepatitis C and developing a screening system for a remedy for human hepatitis C.

All publications cited herein are incorporated herein by reference in their entirety. Furthermore, it is apparent for persons skilled in the art that various modifications and changes of the invention may be made without departing from the technical spirit of the appended claims and the scope of the invention. The present invention is intended to further encompass such modifications and changes.

## Claims

1. A transgenic hepatitis C model animal, having the full-length DNA of the hepatitis C virus incorporated therein, and being capable of expressing the full-length HCV gene.

2. The hepatitis C model animal of claim 1, wherein the animal is a mouse.

3. The hepatitis C model animal of claim 1 or 2, wherein the full-length DNA of the hepatitis C virus is introduced so that DNA is expressed by switching expression.

4. The hepatitis C model animal of claim 3, wherein a means to cause the expression of the full-length DNA of hepatitis C virus by switching expression comprises causing a stuffer gene flanked by loxP sequences to be present between the full-length DNA of the hepatitis C virus and a promoter.

5. The hepatitis C model animal of any one of claims 1 to 4, which is a mammal obtained by developing ontogenetically an ES cell (embryonic stem cell) wherein the full-length DNA of the hepatitis C virus is introduced through a chimeric embryo, or the offsprings thereof, and which carries the above full-length HCV gene in the chromosome of the somatic cell and the germ cell.

6. A method of generating a hepatitis C model animal carrying a full-length HCV gene, comprising introducing a vector containing the full-length DNA of hepatitis C virus into an ES cell, and developing ontogenetically a chimeric embryo having the ES cell as a component in a pseudo-parent.

7. The generation method of claim 6, wherein the animal is a mouse.

8. The generation method of claim 6 or 7, wherein the full-length DNA of hepatitis C virus is introduced so that it is expressed by switching expression.

9. The generation method of claim 8, wherein a means to cause the expression of the full-length DNA of the hepatitis C virus by switching expression comprises causing a stuffer gene flanked by loxP sequences to be present between the full-length DNA of the hepatitis C virus and a promoter.

10. A method of screening for a remedy for a hepatitis C virus-related disease or a substance suppressing the proliferation of hepatitis C .virus, comprising the following steps of:
(a) administering a test substance to the animal of any one of claims I to 5,
(b) measuring the hepatitis C virus level in the above animal, and
(c) selecting a substance that reduces hepatitis C virus level compared with that in a case wherein a test substance is not administered.

11. The hepatitis C model animal of any one of claims 1 to 5, wherein an immunodeficient state is introduced.
